# EUROPEAN PATENT APPLICATION

(11) **EP 1 908 768 A1**
(43) Date of publication of application: **09.04.2008**
(21) Application number: 06291556.6
(22) Date of filing: 05.10.2006
(51) Int. Cl.: C07H 11/00, C07H 3/06, A61K 31/702, A61P 7/02

(54) **Anticoagulant compounds**

(71) Applicant: Endotis Pharma, 59120 Loos (FR)
(72) Inventor: Petitou, Maurice, 75013 Paris (FR); Dubreucq, Guy, 59000 Lille (FR); Querolle, Olivier, 91300 Massy (FR); Zameo, Sandrine, 92340 Bourg-la-Reine (FR)
(74) Representative: Howard, Paul Nicholas

(57) **Abstract**

The present invention is concerned with anticoagulants (*i.e.* substances that stop blood from clotting). More specifically, the present invention is concerned with orally available antithrombic oligosaccharides.

## Description

### TECHNICAL FIELD

The present invention is concerned with anticoagulants (i.e. substances that stop blood from clotting). More specifically, the present invention is concerned with orally available antithrombic oligosaccharides.

### BACKGROUND ART

Heparin is an anticoagulant and is a natural sulphated polysaccharide that belongs to the family of glycosaminoglycans. Heparin acts as a controlling agent to prevent massive clotting of blood and, hence, runaway clot formation. The anticoagulant activity of heparin is reflected by its ability to accelerate the inhibition of several proteases in the blood-coagulation cascade including factor Xa and thrombin.

Heparin and heparin derived drugs inhibit the activity of factor Xa by attaching to a specific binding domain of antithrombin (AT). Once the heparin or heparin derived drugs are attached to the specific binding domain of antithrombin, they induce a conformational change in antithrombin (AT). It is the conformational change in AT that inhibits the activity of factor Xa. Investigations have shown that the lowest structural element that is capable of significantly binding AT, and inhibiting factor Xa, is a pentasaccharide.

Saccharides capable of binding to AT can be seen in European Patent 0 649 854, wherein a pentasaccharide chain is said to be particularly advantageous at inhibiting factor Xa. Oligosaccharides capable of inhibiting thrombin, by binding to AT, are also disclosed in WO 98/03554 and WO 99/36443.

Additionally, US patents US 4,841,041 and US 6,670,338 disclose pentasaccharides that have antithrombotic activity and anti-factor Xa activity. These pentasaccharides are said not to inactivate thrombin via inhibition of AT.

There are, however, problems associated with the use of heparins, which can be overcome by using low molecular weight heparins (LMWHs) that have improved pharmacokinetic properties (*e.g.*. longer half-lives) relative to unfractionated heparins. Despite the pharmacokinetic advantages associated with the use of LMWHs, due to a lack of absorption when administered orally they can only be administered parenterally.

There therefore remains a need for the production of an anticoagulant that can be orally administered. Ideally, such anticoagulants should be stable under acidic conditions, such as those found in the stomach. The present invention aims to produce oligosaccharide derivatives that act as anticoagulants and possess improved properties, such that they are capable of oral administration.

### DISCLOSURE OF THE INVENTION

According to one aspect of the present invention, there is provided a compound, a salt, solvate or prodrug thereof comprising a pentasaccharide that is capable of acting as an anticoagulant and inhibiting factor Xa.

### Pentasaccharide

Anticoagulants in the heparin family, such as LMWHs, are negatively charged and hydrophilic, which causes restrictions on their clinical use. Anticoagulants, such as LMWHs, typically have a low oral bioavailability which makes them unsuitable for oral administration.

The compounds of the present invention contain a reduced number of sulfate groups, while retaining a pharmacological effect (*i.e.* anticoagulant activity). The hydrophilicity problems that are encountered when using anticoagulants in the heparin family have been overcome by substituting hydroxyl groups with hydrophobic groups. These substitutions reduce the hydrophilicity of the molecule making it more suitable for oral administration.

The oligosaccharides of the present invention are of Formula (I): 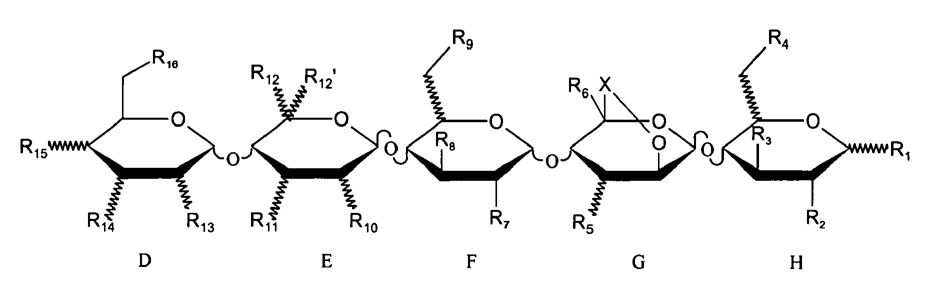 wherein:
R₂, R₇, R₈ and R₁₆ are independently selected from the group consisting of: OSO₃H and NHSO₃H;
R₆ and R₁₂ are each COOH;
R₁, R₃, R₄, R₅, R₉, R₁₀, R₁₁, R₁₃, R₁₄ and R₁₅ are independently selected from the group consisting of: OH, OSO₃H, NH₂, O-alkyl, O-acyl, O-alkenyl, O-alkynyl, O-aryl, O-heteroaryl, O-heterocyclyl, O-aminoalkyl, O-alkylaryl, O-alkylheteroaryl, O-alkylheterocyclyl;
provided at least one of R₃, R₄, R₉, R₁₀, R₁₃, R₁₄ and R₁₅ is independently selected from the group consisting of: NH₂, O-(C₄₋₃₀-alkyl), O-(C₄₋₃₀-acyl), O-alkenyl, O-alkynyl, O-aryl, O-heteroaryl, O-heterocyclyl, O-aminoalkyl, O-alkylaryl, O-alkylheteroaryl, O-alkylheterocyclyl;
R_{12'} is selected from the group consisting of: H and alkyl;
X is selected from the group consisting of: CH₂ and CH₂CH₂; and
wherein any of R₃, R₄, R₉, R₁₀ R₁₃, R₁₄ and R₁₅ are independently optionally substituted with one or more groups, preferably one, two or three of the groups, independently selected from alkyl, alkoxyalkyl, alkoxyaryl, alkynyl, heteroaryl, aryl, arylalkyl, alkaryl, COOH, COOalkyl, SH, S-alkyl, SO₂H, SO₂alkyl, SO₂aryl SO₂alkaryl, P(OH)(O)₂, halo, haloalkyl, perhaloalkyl, OH, O-alkyl, =O, NH₂, =NH, NHalkyl, N(alkyl)₂, =Nalkyl, NHC(O)alkyl, C(O)NH₂, C(O)NHalkyl, C(O)N(alkyl)₂, C(O)NHaryl, NO₂, CN, SO₂, SO₂NH₂, C(O)H, C(O)alkyl;
or a salt, solvate or prodrug thereof.

More preferably, the oligosaccharide of the present invention is of Formula (II):

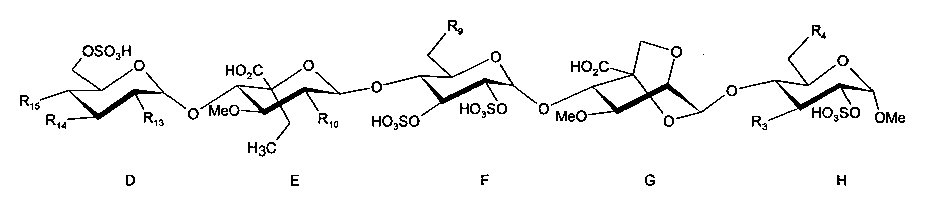

In a preferred aspect of the present invention, the group R₃ is OSO₃H.

In another preferred aspect of the present invention, the groups R₁, R₅ and R₁₁ are each O-alkyl.

In another preferred aspect of the present invention, the group R₁₀ is OCH₃.

In another preferred aspect of the present invention, the group R₁₃ is NH₂.

In another preferred aspect of the present invention, the groups R₄, R₉, R₁₄ and R₁₅ are each OH.

In another aspect of the present invention, the groups R₃, R₄, R₉, R₁₀, R₁₃, R₁₄ and R₁₅ are independently selected from the group consisting of: OH, OSO₃H, NH₂, O-(C₄₋₃₀-alkyl), O-(C₄₋₃₀-acyl), O-alkenyl, O-alkynyl, O-aryl, O-heteroaryl, O-heterocyclyl, O-aminoalkyl, O-alkylaryl, O-alkylheteroaryl, O-alkylheterocyclyl;
wherein any of R₃, R₄, R₉, R₁₀, R₁₃, R₁₄ and R₁₅ are independently optionally substituted with one or more groups, preferably one, two or three of the groups, independently selected from alkyl, alkoxyalkyl, alkoxyaryl, alkynyl, heteroaryl, aryl, arylalkyl, alkaryl, COOH, COOalkyl, SH, S-alkyl, SO₂H, SO₂alkyl, SO₂aryl SO₂alkaryl, P(OH)(O)₂, halo, haloalkyl, perhaloalkyl, OH, O-alkyl, =O, NH₂, =NH, NHalkyl, N(alkyl)₂, =Nalkyl, NHC(O)alkyl, C(O)NH₂, C(O)NHalkyl, C(O)N(alkyl)₂, NO₂, CN, SO₂, SO₂NH₂, C(O)H, C(O)alkyl and C(O)NHaryl.

Preferably, the groups R₃, R₄, R₉, R₁₀, R₁₃, R₁₄ and R₁₅ are independently selected from: OH, OSO₃H, NH₂, O-(C₄₋₃₀-alkyl), O-(C₄₋₃₀-acyl O-heterocyclyl, O-aryl, O-alkylaryl;
wherein any of R₃, R₄, R₉, R₁₀, R₁₃, R₁₄ and R₁₅ are independently optionally substituted with one or more groups, preferably one, two or three of the groups, independently selected from halo, haloalkyl, perhaloalkyl, OH, O-alkyl, =O, alkyl, alkoxyalkyl, alkoxyaryl, alkynyl, arylalkyl, alkaryl, heteroaryl and aryl.

More preferably, the groups R₃, R₄, R₉, R₁₀, R₁₃, R₁₄ and R₁₅ are independently selected from: O-butyl, nonanoyl, (4-*tert*-butyl)benzyloxy, 3-cyclopentylpropanoyl, hexanoyl, 2,2-dimethylpropyloxy, 4-chlorobenzyloxy, OH and deoxycholoyl.

In a preferred aspect of the present invention, the groups R₃, R₄, R₉, R₁₀, R₁₃, R₁₄ and R₁₅ are each O-butyl.

In another preferred aspect of the present invention, the groups R₃, R₄, R₉, R₁₀, R₁₃, R₁₄ and R₁₅ are each nonanoyl.

In a preferred aspect of the present invention, the groups R₃, R₁₀, R₁₃, R₁₄ and R₁₅ are each (4-tert-butyl)benzyloxy.

In another preferred aspect of the present invention, the groups R₃, R₁₀, R₁₃, R₁₄ and R₁₅ are each hexanoyl.

In another preferred aspect of the present invention, the groups R₃, R₁₀, R₁₃, R₁₄ and R₁₅ are each 4-chlorobenzyloxy).

In another preferred aspect of the present invention, the groups R₃, R₁₀, R₁₃, R₁₄ and R₁₅ are each OH.

In a preferred aspect of the present invention, the groups R₄ and R₉ are each 3-cyclopentylpropanoyl.

In another preferred aspect of the present invention, the groups R₄ and R₉ are each 2,2-dimethylpropyloxy.

In a preferred aspect of the present invention, the groups R₄ and R₉ are each OH.

In a preferred aspect of the present invention, the groups R₄ and R₉ are each deoxycholoyl.

In the present specification, the groups -COOH, -OSO₃H and -NHSO₃H are represented in their acid form. It will be understood the representation in their acid form also extends to their salt form. In a preferred embodiment these groups are in their salt form, more preferably in their sodium salt form.

It will be appreciated that the pentasaccharide can exist in a variety of stereochemical forms, which will be apparent to one skilled in the art. Positions of variable stereochemistry include those indicated with wavy lines. Except where specifically indicated, the present invention extends to all such stereochemical forms.

Advantageously, the G monosaccharide unit of the oligosaccharide has the following conformation:

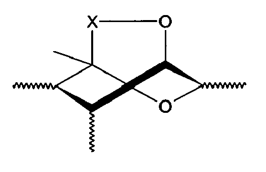

Preferably, the D, E, F and H monosaccharide units of the oligosaccharide have the D-gluco stereochemistry:

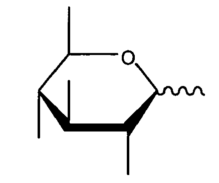

Additionally, it is preferred that the G monosaccharide unit of the oligosaccharide has the following stereochemistry:

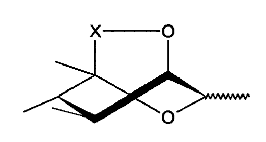

In a preferred aspect of the invention, the groups R₁, R₅ and R₁₁ are each OMe.

In another preferred aspect of the invention, the groups R₂, R₇, R₈ and R₁₆ are each OSO₃H.

In another preferred aspect of the invention, the group X is CH₂.

In another preferred aspect of the invention, the group R_{12'} is CH₂CH₃.

For the avoidance of doubt, the present invention extends to any combination of the aforementioned aspects.

In a further aspect of the present invention, a pharmaceutical composition is provided comprising a pentasaccharide, as described in the present invention, and a pharmaceutically acceptable diluent or carrier.

The present invention also provides a method of making a pharmaceutical composition, comprising mixing the pentasaccharide of the present invention with a pharmaceutically acceptable diluent or carrier.

In a further aspect of the present invention, there is provided use of a pentasaccharide, as described in the present invention, in therapy.

In another aspect of the invention, there is provided the use of a pentasaccharide, as defined in the present invention, in the manufacture of a medicament for the treatment of a blood clotting disorder.

The present invention also provides a method of treating a blood clotting disorder in a human or animal subject comprising administering to the human or animal subject a therapeutically effective amount of a pentasaccharide, as defined in the present invention.

In another aspect of the invention, the medicament as described above, for oral administration. Preferably, the method of treatment also involves oral administration.

Preferably, the blood clotting disorder is selected from: deep vein thromboembolism including deep vein thrombosis and pulmonary embolism, post surgical prophylaxis of deep venous thrombosis, coronary syndromes, myocardial infarcation and stroke.

### DEFINITIONS

### Pharmaceutical compositions

The compounds of the present invention may also be present in the form of pharmaceutically acceptable salts. For use in medicine, the salts of the compounds of this invention refer to non-toxic "pharmaceutically acceptable salts." FDA approved pharmaceutically acceptable salt forms (Gould, P. L. International J. Pharm., 1986, 33, 201-217; Berge, S.M. et al. J. Pharm. Sci., 1977, 66(1), 1-19) include pharmaceutically acceptable acidic/anionic or basic/cationic salts.

Pharmaceutically acceptable salts of the acidic or basic compounds of the invention can of course be made by conventional procedures, such as by reacting the free base or acid with at least a stoichiometric amount of the desired salt-forming acid or base.

Pharmaceutically acceptable salts of the acidic compounds of the invention include salts with inorganic cations such as sodium, potassium, calcium, magnesium, zinc, and ammonium, and salts with organic bases. Suitable organic bases include N-methyl-D-glucamine, arginine, benzathine, diolamine, olamine, procaine and tromethamine.

Pharmaceutically acceptable salts of the basic compounds of the invention include salts derived from organic or inorganic acids. Suitable anions include acetate, adipate, besylate, bromide, camsylate, chloride, citrate, edisylate, estolate, fumarate, gluceptate, gluconate, glucuronate, hippurate, hyclate, hydrobromide, hydrochloride, iodide, isethionate, lactate, lactobionate, maleate, mesylate, methylbromide, methylsulfate, napsylate, nitrate, oleate, pamoate, phosphate, polygalacturonate, stearate, succinate, sulfate, sulfosalicylate, tannate, tartrate, terephthalate, tosylate and triethiodide. Hydrochloride salts are particularly preferred.

The invention also comprehends derivative compounds ("pro-drugs") which are degraded *in vivo* to yield the species of Formula (I). Pro-drugs are usually (but not always) of lower potency at the target receptor than the species to which they are degraded. Pro-drugs are particularly useful when the desired species has chemical or physical properties, which make its administration difficult or inefficient. For example, the desired species may be only poorly soluble, it may be poorly transported across the mucosal epithelium, or it may have an undesirably short plasma half-life. Further discussion of pro-drugs may be found in Stella, V. J. et al. "Prodrugs", Drug Delivery Systems, 1985, 112-176, Drugs, 1985, 29, 455-473 and "Design of Prodrugs", ed. H. Bundgaard, Elsevier, 1985.

The compounds described in the claims having an amino group may be derivatised with a ketone or an aldehyde such as formaldehyde to form a Mannich base. This will hydrolyse with first order kinetics in aqueous solution. In addition, the compounds described in the claims having one or more free hydroxy groups may be esterified in the form of a pharmaceutically acceptable ester. This may be convertible, by solvolysis, under physiological conditions to the compounds of the present invention having free hydroxy groups.

Thus, in the methods of treatment of the present invention, the term "administering" shall encompass the treatment of the various disorders described with the compound specifically disclosed or with a compound which may not be specifically disclosed, but which converts to the specified compound *in vivo* after administration to the subject.

It is anticipated that the compounds of the invention can be administered by oral or parenteral routes, including intravenous, intramuscular, intraperitoneal, subcutaneous, transdermal, rectal and topical administration, and inhalation. Oral administration of the compounds of the present invention is particularly preferred.

For oral administration, the compounds of the invention will generally be provided in the form of tablets or capsules or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate and lactose. Corn starch and alginic acid are suitable disintegrating agents. Binding agents may include starch and gelatine. The lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatine capsules in which the active ingredient is mixed with a solid diluent and soft gelatine capsules wherein the active ingredient is mixed with water or an oil such as peanut oil, liquid paraffin or olive oil.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The pharmaceutical compositions of the present invention may, in particular, comprise more than one compound (multiple) of the present invention, *e.g.*, two or more compounds. The invention also provides a pharmaceutical preparation or system, comprising (a) a first compound, which is a compound of the invention; and (b) a second pharmaceutical compound. Said multiple compounds of the invention or said first and second compounds are formulated either in admixture or as separate compositions, *e.g.* for simultaneous though separate, or for sequential administration (see below).

### Modes of Administration

The compounds of the present invention can be delivered directly or in pharmaceutical compositions containing excipients (see above), as is well known in the art. The present methods of treatment involve administration of a therapeutically effective amount of a compound of the present invention to a subject.

The term "therapeutically effective amount" or "therapeutically effective dose" as used herein refers to an amount of a compound according to the present invention needed to: treat; ameliorate; prevent the targeted disease condition; exhibit a detectable therapeutic or preventative effect; prolong survival of a patient. Toxicity and therapeutic efficacy of such molecules can be determined by standard pharmaceutical procedures in cell cultures or experimental animals, *e.g.*, by determining the LD50 (the dose lethal to 50% of the population) and the ED50 (the dose therapeutically effective in 50% of the population). The dose ratio of toxic to therapeutic effects is the therapeutic index, which can be expressed as the ratio LD50/ED50. Agents that exhibit high therapeutic indices are preferred.

The therapeutically effective amount or therapeutically effective dose is the amount of the compound or pharmaceutical composition that will elicit the biological or medical response of a tissue, system, animal, or human that is being sought by the researcher, veterinarian, medical doctor, or other clinician. For example, anticoagulant activity and treatment of blood clotting disorders, *e.g.*, deep vein thromboembolism including deep vein thrombosis and pulmonary embolism, post surgical deep venous thrombosis, coronary syndromes, myocardial infarcation, stroke, etc.

Dosages preferably fall within a range of circulating concentrations that includes the ED50 with little or no toxicity. Dosages may vary within this range depending upon the dosage form employed and/or the route of administration utilised. The exact formulation, route of administration, dosage, and dosage interval should be chosen according to methods known in the art, in view of the specifics of a patient's condition.

Dosage amount and interval may be adjusted individually to provide plasma levels of the active moiety that are sufficient to achieve the desired effects, *i.e.,* minimal effective concentration (MEC). The MEC will vary for each compound but can be estimated from, for example, *in vitro* data and animal experiments. Dosages necessary to achieve the MEC will depend on individual characteristics and route of administration. In cases of local administration or selective uptake, the effective local concentration of the drug may not be related to plasma concentration.

In general, the therapeutically effective dose/amount can be estimated by using conventional methods and techniques that are known in the art. Initial doses used in animal studies (*e.g.* non-human primates, mice, rabbits, dogs, or pigs) may be based on effective concentrations established in cell culture assays. The animal model may also be used to determine the appropriate concentration range and route of administration. Such information can then be used to determine useful doses and routes for administration in human patients.

The specific dosage level required for any particular patient will depend on a number of factors, including severity of the condition being treated, the route of administration, the general health of the patient (*i.e.* age, weight and diet), the gender of the patient, the time and frequency of administration, judgement of the prescribing physician and tolerance/response to therapy. In general, however, the daily dose (whether administered as a single dose or as divided doses) will be in the range 0.01 to 500 mg per day, more usually from 0.1 to 50 mg per day, and most usually from 1 to 10 mg per day. Alternatively, dosages can be administered per unit body weight and, in this instance, a typical dose will be between 0.001 mg/kg and 3 mg/kg, especially between 0.01 mg/kg and 0.2 mg/kg, between 0.02 mg/kg and 0.1 mg/kg.

An effective and convenient route of administration and an appropriate formulation of the compounds of the invention in pharmaceutical compositions (see above) may also be readily determined by routine experimentation. Various formulations and drug delivery systems are available in the art (see, *e.g.*, Gennaro AR (ed.). Remington: The Science and Practice of Pharmacy. Lippincott Williams & Wilkins. 21st edition. July 3, 2005 and Hardman JG, Limbird LE, Alfred G. Gilman AG. Goodman & Gilman's The Pharmacological Basis of Therapeutics. McGraw-Hill; 10th edition. August 13, 2001).

As mentioned above, suitable routes of administration may, for example, include vaginal, rectal, intestinal, oral, nasal (intranasal), pulmonary or other mucosal, topical, transdermal, ocular, aural, and parenteral administration.

An advantage of the compounds of the present invention is that they are particularly suitable for oral administration.

Primary routes for parenteral administration include intravenous, intramuscular, and subcutaneous administration. Secondary routes of administration include intraperitoneal, intra-arterial, intra-articular, intracardiac, intracisternal, intradermal, intralesional, intraocular, intrapleural, intrathecal, intrauterine, and intraventricular administration. The indication to be treated, along with the physical, chemical, and biological properties of the drug, dictate the type of formulation and the route of administration to be used, as well as whether local or systemic delivery would be preferred.

The present compositions may, if desired, be presented in a pack or dispenser device containing one or more unit dosage forms containing the active ingredient. Such a pack or device may, for example, comprise metal or plastic foil, such as a blister pack, or glass and rubber stoppers such as in vials. The pack or dispenser device may be accompanied by instructions for administration. Compositions comprising an agent of the invention formulated in a compatible pharmaceutical carrier may also be prepared, placed in an appropriate container, and labelled for treatment of an indicated condition.

### Chemical Definitions

Formulaic representation of apparent orientation of a functional group is not necessarily intended to represent actual orientation. Thus, for example, a divalent amide group represented as C(O)NH is also intended to cover NHC(O).

In the interests of simplicity, terms which are normally used to refer to monovalent groups (such as "alkyl" or "alkenyl") are also used herein to refer to divalent, trivalent or tetravalent bridging groups which are formed from the corresponding monovalent group by the loss of one or more hydrogen atom(s). Whether such a term refers to a monovalent group or to a polyvalent group will be clear from the context. Where a polyvalent bridging group is formed from a cyclic moiety, the linking bonds may be on any suitable ring atom, according to the normal rules of valency.

Where any particular moiety is substituted, for example an imidazole group comprising a substituent on the heteroaryl ring, unless specified otherwise, the term "substituted" contemplates all possible isomeric forms. For example, substituted imidazole includes all of the following permutations:

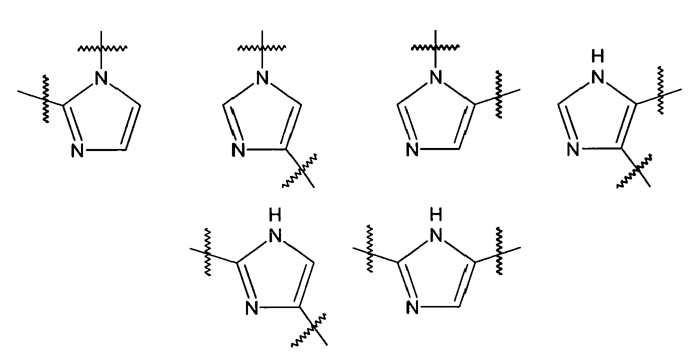

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. The permissible substituents can be one or more and the same or different for appropriate organic compounds. For purposes of this invention, heteroatoms such as nitrogen may have hydrogen substituents and/or any permissible substituents of organic compounds described herein which satisfy the valencies of the heteroatoms. This invention is not intended to be limited in any manner by the permissible substituents of organic compounds.

The terms "comprising" and "comprises" means "including" as well as "consisting" e.g. a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

"Optional" or "optionally" means that the subsequently described event of circumstances may or may not occur, and that the description includes instances where said event or circumstance occurs and instances in which it does not.

"May" means that the subsequently described event of circumstances may or may not occur, and that the description includes instances where said event or circumstance occurs and instances in which it does not.

Where the compounds according to this invention have at least one chiral centre, they may accordingly exist as enantiomers. Where the compounds possess two or more chiral centres, they may additionally exist as diastereomers. Where the processes for the preparation of the compounds according to the invention give rise to mixture of stereoisomers, these isomers may be separated by conventional techniques such as preparative chromatography. The compounds may be prepared in racemic form or individual enantiomers may be prepared by standard techniques known to those skilled in the art, for example, by enantiospecific synthesis or resolution, formation of diastereomeric pairs by salt formation with an optically active acid, followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column. It is to be understood that all such isomers and mixtures thereof are encompassed within the scope of the present invention.

Where a group comprises two or more moieties defined by a single carbon atom number, for example, C₂₋₂₀-alkoxyalkyl, the carbon atom number indicates the total number of carbon atoms in the group.

As used herein, the term "heteroatom" includes N, O, S, P, Si and halogen (including F, Cl, Br and I). In the context of a hydrocarbon chain interrupted by one or more heteroatoms, the term "heteroatoms" includes N, O and S.

The term "halogen" or "halo" is used herein to refer to any of fluorine, chlorine, bromine and iodine. Most usually, however, halogen substituents in the compounds of the invention are chlorine, bromine and fluorine substituents. Groups such as halo(alkyl) include mono-, di-, tri- and per-halo substituted alkyl groups. Moreover, the halo substitution may be at any position in the alkyl chain. "Perhalo" means completely halogenated, e.g., trihalomethyl and pentachloroethyl.

As used herein, the term "alkyl" refers to a cyclic, straight or branched saturated monovalent hydrocarbon radical, having the number of carbon atoms as indicated. For example, the term "C₁₋₃₀-alkyl" includes C₁, C₂, C₃, C₄, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, and C₃₀ alkyl groups. By way of non-limiting example, suitable alkyl groups include methyl, ethyl, propyl, *iso*-propyl, butyl, *iso*-butyl, *tert*-butyl, pentyl, hexyl, octyl, nonyl, dodecyl, eicosyl, cyclopropyl, cyclobutyl, cyclopentyl, cyclohexyl, methylcyclohexyl, dimethylcyclohexyl, trimethylcyclohexyl, cycloheptyl, cyclooctyl, cyclononyl, cyclododecyl, spiroundecyl, bicyclooctyl and adamantyl, cyclopropylmethyl, cyclopropylethyl, cyclobutylmethyl, cyclobutylethyl, cyclopentylmethyl, cyclopentylethyl, cyclohexylmethyl, cyclohexylethyl, cyclohexylpropyl, cyclohexylbutyl, methylcyclohexylmethyl, dimethylcyclohexylmethyl, trimethylcyclohexylmethyl, cycloheptylmethyl, cycloheptylethyl, cycloheptylpropyl, cycloheptylbutyl and adamantylmethyl. Preferred ranges of alkyl groups of the present invention are: C₁₋₃₀-alkyl, C₂₋₂₈-alkyl, C₃₋₂₆-alkyl, C₄₋₂₄-alkyl, C₄₋₂₂-alkyl, C₅₋₂₀-alkyl, C₅₋₁₈-alkyl, C₆₋₁₆-alkyl, C₇₋₁₄-alkyl and C₈₋₁₂-alkyl. Preferred ranges in cycloalkyl groups are: C₄₋₃₀, C₄₋₂₀, C₄₋₁₅ and C₅₋₁₃.

As used herein, the term "alkenyl" refers to a cyclic, straight or branched unsaturated monovalent hydrocarbon radical, having the number of carbon atoms as indicated, and the distinguishing feature of a carbon-carbon double bond. For example, the term "C₂₋₃₀-alkenyl" includes C₂, C₃, C₄, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, and C₃₀ alkenyl groups. By way of non-limiting example, suitable alkenyl groups include ethenyl, propenyl, butenyl, penentyl, hexenyl, octenyl, nonenyl, dodecenyl and eicosenyl, wherein the double bond may be located anywhere in the carbon chain. Preferred ranges of alkenyl groups of the present invention are: C₂₋₃₀-alkenyl, C₂₋₂₈-alkenyl, C₃₋₂₆-alkenyl, C₄₋₂₄-alkenyl, C₅₋₂₂-alkenyl, C₅₋₂₀-alkenyl, C₆₋₁₈-alkenyl, C₆₋₁₆-alkenyl, C₇₋₁₄-alkenyl and C₈₋₁₂-alkenyl. Preferred ranges in cycloalkenyl groups are: C₄₋₃₀, C_{4-20,} C₅₋₁₅ and C₆₋₁₃.

As used herein, the term "alkynyl" refers to a cyclic, straight or branched unsaturated monovalent hydrocarbon radical, having the number of carbon atoms as indicated, and the distinguishing feature of a carbon-carbon triple bond. For example, the term "C₂₋₃₀ alkynyl" includes C₂, C₃, C₄, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, and C₃₀ alkynyl groups. By way of non-limiting example, suitable alkynyl groups include ethynyl, propynyl, butynyl, penynyl, hexynyl, octynyl, nonynyl, dodycenyl and eicosynyl, wherein the triple bond may be located anywhere in the carbon chain. Preferred ranges of alkynyl groups of the present invention are: C₂₋₃₀-alkynyl, C₂₋₂₈-alkynyl, C₃₋₂₆-alkynyl, C₄₋₂₄-alkynyl, C₄₋₂₂-alkynyl, C₅₋₂₀-alkynyl, C₅₋₈-alkynyl, C₆₋₁₆-alkynyl, C₇₋₁₄-alkynyl and C₈₋₁₂-alkynyl. Preferred ranges in cycloalkenyl groups are: C₈₋₃₀, C₉₋₂₀, C₅₋₁₅ and C_{10-13.}

Alkoxy refers to the group "alkyl-O-", where alkyl is as defined above. By way of non-limiting example, suitable alkoxy groups include methoxy, ethoxy, propoxy, butoxy, pentoxy and hexoxy.

As used herein, the term "alkoxyalkyl" refers to an alkyl group having an alkoxy substituent. Binding is through the alkyl group. The alkyl moiety may be cyclic, straight or branched. The alk and alkyl moieties of such a group may be substituted as defined above, with regard to the definition of alkyl. By way of non-limiting example, suitable alkoxyalkyl groups include methoxymethyl, methoxyethyl, ethoxymethyl, ethoxyethyl, methoxypropyl and ethoxypropyl.

As used herein, the term "alkoxyaryl" refers to an aryl group having an alkoxy substituent. Binding is through the aryl group. The alkoxy and aryl moieties of such a group may be substituted as defined herein, with regard to the definitions of alkoxy and aryl. The alkyl moiety may be cyclic, straight or branched. By way of non-limiting example, suitable alkoxyaryl groups include methoxyphenyl, ethoxyphenyl, dimethoxyphenyl and trimethoxyphenyl.

As used herein, the term "aryl" refers to monovalent aromatic carbocyclic radical having one, two, three, four, five or six rings, preferably one, two or three rings, which may be fused or bicyclic. Preferably, the term "aryl" refers to an aromatic monocyclic ring containing 6 carbon atoms, which may be substituted on the ring with 1, 2, 3, 4 or 5 substituents as defined herein; an aromatic bicyclic or fused ring system containing 7, 8, 9 or 10 carbon atoms, which may be substituted on the ring with 1, 2, 3, 4, 5, 6, 7, 8 or 9 substituents as defined herein; or an aromatic tricyclic ring system containing 10, 11, 12, 13 or 14 carbon atoms, which may be substituted on the ring with 1, 2, 3, 4, 5, 6, 7, 8, 9, 10, 11, 12 or 13 substituents as defined herein. By way of non-limiting example, suitable aryl groups include phenyl, biphenyl, binaphthyl, indanyl, phenanthryl, fluoryl, flourenyl, stilbyl, benzylphenanthryl, acenaphthyl, azulenyl, phenylnaphthyl, benzylfluoryl, tetrahydronaphthyl, perylenyl, picenyl, chrysyl, pyrenyl, tolyl, chlorophenyl, dichlorophenyl, trichlorophenyl, methoxyphenyl, dimethoxyphenyl, trimethoxyphenyl, fluorophenyl, difluorophenyl, trifluorophenyl, nitrophenyl, dinitrophenyl, trinitrophenyl, aminophenyl, diaminophenyl, triaminophenyl, cyanophenyl, chloromethylphenyl, tolylphenyl, xylylphenyl, chloroethylphenyl, trichloromethylphenyl, dihydroindenyl, benzocycloheptyl and trifluoromethylphenyl. Preferred ranges of aryl groups of the present invention are: C₆₋₂₅-aryl, C₆₋₂₃-aryl, C₆₋₂₀-aryl, C₆₋₁₈-aryl, C₆₋₁₅-aryl, C₆₋₁₂-aryl, C₆₋₁₀-aryl, C₆₋₉-aryl, C₆₋₈-aryl and C₆₋₇-aryl.

The term "heteroaryl" refers to a monovalent unsaturated aromatic heterocyclic radical having one, two, three, four, five or six rings, preferably one, two or three rings, which may be fused or bicyclic. Preferably, "heteroaryl" refers to an aromatic monocyclic ring system containing five members of which at least one member is a N, O or S atom and which optionally contains one, two or three additional N atoms, an aromatic monocyclic ring having six members of which one, two or three members are a N atom, an aromatic bicyclic or fused ring having nine members of which at least one member is a N, O or S atom and which optionally contains one, two or three additional N atoms or an aromatic bicyclic ring having ten members of which one, two or three members are a N atom. By way of non-limiting example, suitable heteroaryl groups include furanyl, pryingly, pyridyl, phthalimido, thiophenyl, pyrrolyl, imidazolyl, pyrazolyl, thiazolyl, isothiazolyl, oxazolyl, oxadiazolyl, pyronyl, pyrazinyl, tetrazolyl, thionaphthyl, benzofuranyl, isobenzofuryl, indolyl, oxyindolyl, isoindolyl, indazolyl, indolinyl, azaindolyl, isoindazolyl, benzopyranyl, coumarinyl, isocoumarinyl, quinolyl, isoquinolyl, cinnolinyl, quinazolinyl, pyridopyridyl, benzoxazinyl, quinoxadinyl, chromenyl, chromanyl, isochromanyl, carbolinyl, thiazolyl, isoxazolyl, isoxazolonyl, isothiazolyl, triazolyl, oxadiazolyl, thiadiazolyl, benzodioxepinyl and pyridazyl. Preferred ranges of heteroaryl groups of the present invention are: C₂₋₃₀-heteroaryl, C₂₋₂₅-heteroaryl, C₂₋₂₀-heteroaryl, C₂₋₁₈-heteroaryl, C₂₋₁₅-heteroaryl, C₂₋₁₂-heteroaryl, C₂₋₁₀-heteroaryl, C₂₋₉-heteroaryl, C₂₋₈-heteroaryl and C₂₋₇-heteroaryl.

The term "heterocyclyl" refers to a saturated or partially unsaturated ring having three members of which at least one member is a N, O or S atom and which optionally contains one additional O atom or additional N atom; a saturated or partially unsaturated ring having four members of which at least one member is a N, O or S atom and which optionally contains one additional O atom or one or two additional N atoms; a saturated or partially unsaturated ring having five members of which at least one member is a N, O or S atom and which optionally contains one additional O atom or one, two or three additional N atoms; a saturated or partially unsaturated ring having six members of which one, two or three members are an N, O or S atom and which optionally contains one additional O atom or one, two or three additional N atoms; a saturated or partially unsaturated ring having seven members of which one, two or three members are an N, O or S atom and which optionally contains one additional O atom or one, two or three additional N atoms; a saturated or partially unsaturated ring having eight members of which one, two or three members are an N, O or S atom and which optionally contains one additional O atom or one, two or three additional N atoms; a saturated or partially unsaturated bicyclic ring having nine members of which at least one member is a N, O or S atom and which optionally contains one, two or three additional N atoms; or a saturated or partially unsaturated bicyclic ring having ten members of which one, two or three members are an N, O or S atom and which optionally contains one additional O atom or one, two or three additional N atoms. Preferably, heterocycles comprising peroxide groups are excluded from the definition of heterocyclyl. By way of non-limiting example, suitable heterocyclyl groups include pyrrolinyl, pyrrolidinyl, dioxolanyl, tetrahydrofuranyl, morpholinyl, imidazolinyl, imidazolidinyl, maleimidyl, pyrazolidinyl, piperidinyl, dihydropyranyl, succinimidyl, tetrahydropyranyl, thiopyranyl, tetrahydrothiopyranyl and piperazinyl. Preferred ranges of heterocyclyl groups of the present invention are: C₂₋₃₀-heterocyclyl, C₂₋₂₅-heterocyclyl, C₂₋₂₀-heterocyclyl, C₂₋₁₈-heterocyclyl, C₂₋₁₅-heterocyclyl, C₂₋₁₂-heterocydyl, C₂₋₁₀-heterocyclyl, C₂₋₉-heterocyclyl, C₂₋₈-heterocyclyl and C₂₋₇-heterocyclyl.

As used herein, the term "alkaryl" and "alkylaryl" refer to an aryl group with an alkyl substituent. Binding is through the aryl group. Such groups have the number of carbon atoms as indicated. The alkyl and aryl moieties of such a group may be substituted as defined herein, with regard to the definitions of alkyl and aryl. The alkyl moiety may be straight or branched. Particularly preferred examples of alkaryl include tolyl, xylyl, butylphenyl, mesityl, ethyltolyl, methylindanyl, methylnaphthyl, methyltetrahydronaphthyl, ethylnaphthyl, dimethylnaphthyl, propylnaphthyl, butylnaphthyl, methylfluoryl and methylchrysyl. Again, preferred ranges of carbon atoms for alkaryl and alkylaryl groups of the present invention are: C₇₋₃₀, C₇₋₂₅, C₇₋₂₀, C₇₋₁₈, C₇₋₁₅, C₇₋₁₂, C₇₋₁₀ and C₇₋₉.

As used herein, the term "arylalkyl" refers to an alkyl group with an aryl substituent. Binding is through the alkyl group. Such groups have the number of carbon atoms as indicated. The aryl and alkyl moieties of such a group may be substituted as defined herein, with regard to the definitions of aryl and alkyl. The alkyl moiety may be straight or branched. Particularly preferred examples of arylalkyl include benzyl, methylbenzyl, ethylbenzyl, dimethylbenzyl, diethylbenzyl, methylethylbenzyl, methoxybenzyl, chlorobenzyl, dichlorobenzyl, trichlorobenzyl, phenethyl, phenylpropyl, diphenylpropyl, phenylbutyl, biphenylmethyl, fluorobenzyl, difluorobenzyl, trifluorobenzyl, phenyltolylmethyl, trifluoromethylbenzyl, bis(trifluoromethyl)benzyl, propylbenzyl, tolylmethyl, fluorophenethyl, fluorenylmethyl, methoxyphenethyl, dimethoxybenzyl, dichlorophenethyl, phenylethylbenzyl, isopropylbenzyl, diphenylmethyl, propylbenzyl, butylbenzyl, dimethylethylbenzyl, phenylpentyl, tetramethylbenzyl, phenylhexyl, dipropylbenzyl, triethylbenzyl, cyclohexylbenzyl, naphthylmethyl, diphenylethyl, triphenylmethyl and hexamethylbenzyl. Similarly, preferred ranges of carbon atoms for arylalkyl groups of the present invention are: C₇₋₃₀, C₇₋₂₅, C₇₋₂₀, C₇₋₁₈, C₇₋₁₅, C₇₋₁₂, C₇₋₁₀ and C₇₋₉.

The term "aminoalkyl" refers to an alkyl group with an amine substituent. Binding is through the alkyl group. Such groups have the number of carbon atoms as indicated above for "alkyl" groups. The alkyl moiety of such a group may be substituted as defined herein, with regard to the definition of alkyl. By way of non-limiting example, suitable aminoalkyl groups include aminomethyl, aminoethyl, aminopropyl, aminobutyl, aminopentyl and aminohexyl.

The term "aminoaryl" refers to an amine group with an aryl substituent. Binding is through the alkyl group. Such groups have the number of carbon atoms as indicated above for "aryl" groups. The aryl moiety of such a group may be substituted as defined herein, with regard to the definition of aryl.

As used herein, the term "acyl" refers to a group of general formula -C(O)-R, wherein R is selected from any one of the following groups: alkyl, alkenyl, alkynyl, aryl, heteroaryl, heterocyclyl, alkylaryl and arylalkyl.

With regard to one or more substituents which are referred to as being optionally substituted within a compound definition, for example, "alkaryl", the substituent may be on either or both of the component moieties, *e.g.*, on the alkyl and/or aryl moieties.

Reference to cyclic systems, *e.g.*, aryl, heteroaryl, *etc*., contemplates monocyclic and polycyclic systems. Such systems comprise fused, non-fused and spiro conformations, such as bicyclooctyl, adamantyl, biphenyl and benzofuran.

The term "monosaccharide" means a sugar molecule having a chain of 3-10 carbon atoms in the form of an aldehyde (aldose) or ketone (ketose). Suitable monosaccharides for use in the invention include both naturally occurring and synthetic monosaccharides. Such monosaccharides include pentoses, such as xylose, arabinose, ribose, lyxose; methyl pentoses (6-deoxyhexoses), such as rhamnose and fructose; hexoses, such as allose, altrose, glucose, gulose, idose, mannose, galactose and talose. Preferred monosaccharides are hexoses.

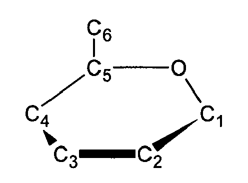

The monosaccharides may be attached to another monosaccharide group at the C₁, C₂, C₃, C₄, C₅ and C₆ position (shown above) to form a glycosyl bond and an oligosaccharide. Typically, a monosaccharide is attached to the C₃, C₄, C₅ and C₆ position through an oxygen atom attached to the C₁ carbon of another monosaccharide, which forms a glycosidic linkage and an oligosaccharide. Oligosaccharides that can be used in the present invention include disaccharides, trisaccharides, tetrasaccharides and pentasaccharides. However, in order to bind to AT, the oligosaccharide of the present invention is a pentasaccharide.

It will be appreciated that ionisable groups may exist in the neutral form shown in formulae herein, or may exist in charged form *e.g.* depending on pH. Thus a carboxylate group may be shown as -COOH, this formula is merely representative of the neutral carboxylate group, and other charged forms are encompassed by the invention (*i.e.* COO⁻).

Similarly, references herein to cationic and anionic groups should be taken to refer to the charge that is present on that group under physiological conditions *e.g.* where a sulphate group -OSO₃H is deprotonated to give the anionic -OSO₃⁻ group, this deprotonation is one that can occur at physiological pH. In addition where a carboxyl group -COOH is deprotonated to give the anionic -COO⁻ group, this deprotonation is one that can occur at physiological pH. Moreover, charged salts of the molecules of the invention are encompassed. Saccharide rings can exist in an open and closed form, while closed forms are shown herein, open forms are also encompassed by the invention.

Certain compounds of the invention exist in various regioisomeric, enantiomeric, tautomeric and diastereomeric forms. It will be understood that the invention comprehends the different regioisomers, enantiomers, tautomers and diastereomers in isolation from each other as well as mixtures.

The counter-ions, which compensate the charged forms of the compounds of the present invention, are pharmaceutically acceptable counter-ions such as hydrogen, or more preferably alkali or alkali-earth metals ions, which include sodium, calcium, magnesium and potassium.

Other 'compound' group definitions will be readily understandable by the skilled person based on the previous definitions and the usual conventions of nomenclature.

It will be appreciated that any optional feature that has been described above in relation to any one aspect of the invention may also be applicable to any other aspect of the invention.

### GENERAL PROCEDURES

The present invention will now be described in more detail, by way of the following non-limiting methods that can be used for synthesising the compounds of the present invention. The skilled person will, however, appreciate that these methods merely illustrate the present invention and in no way restrict its scope.

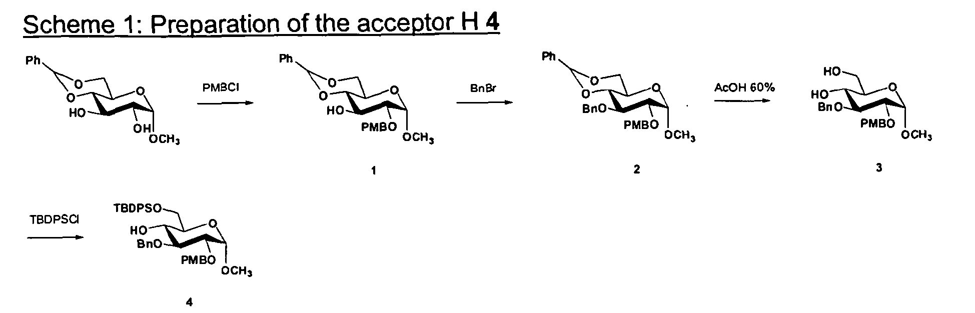

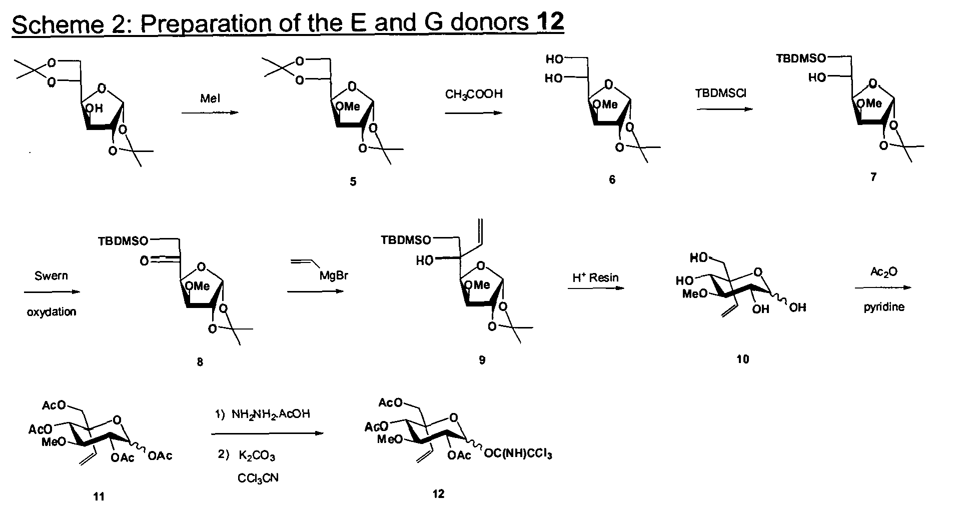

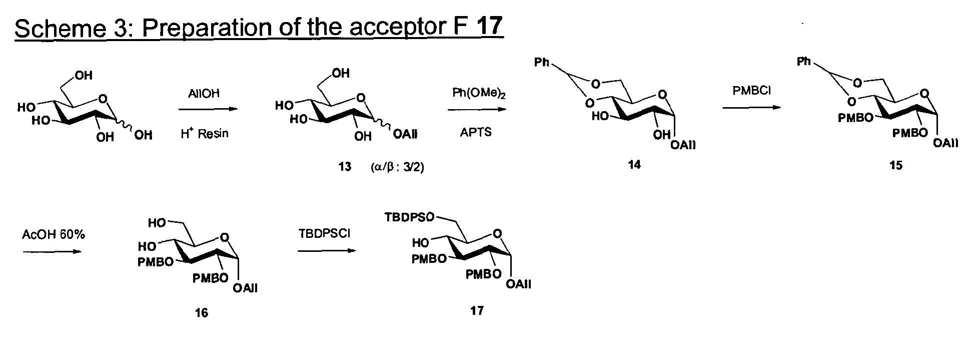

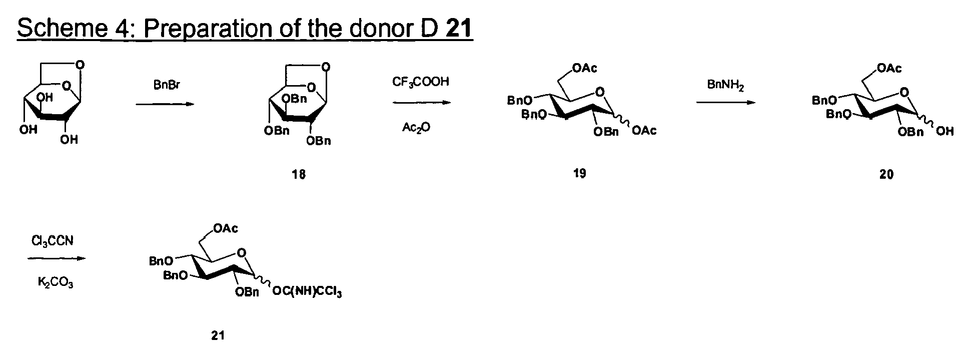

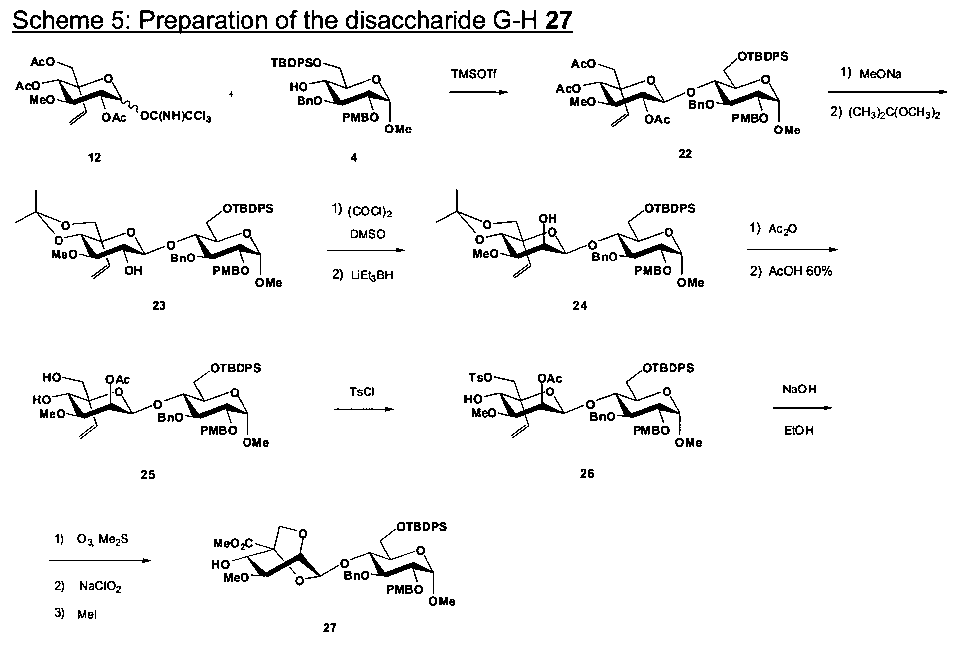

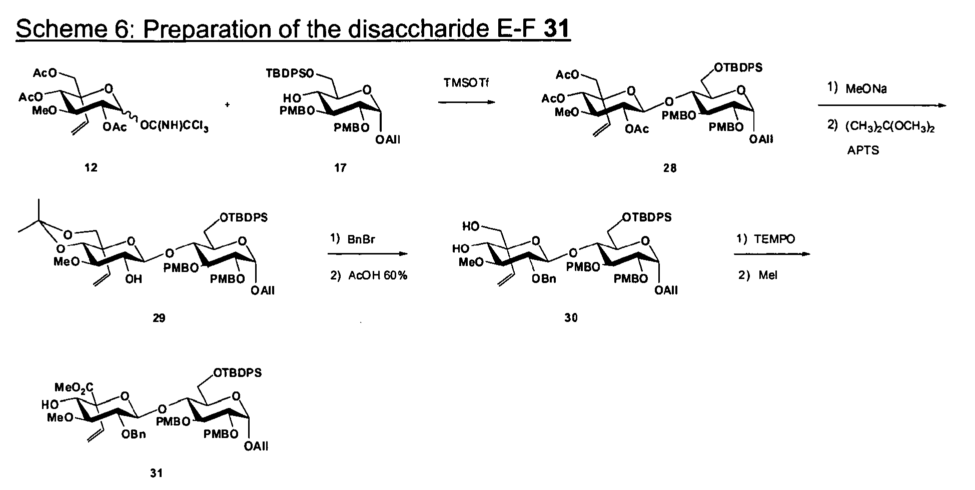

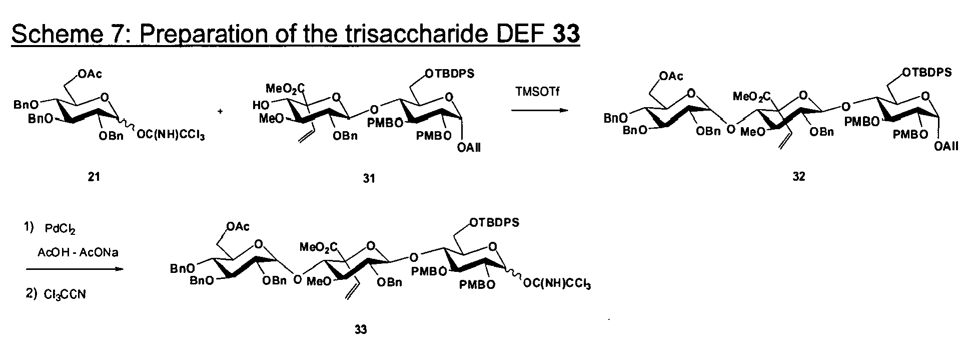

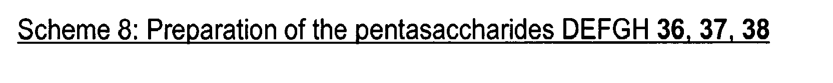

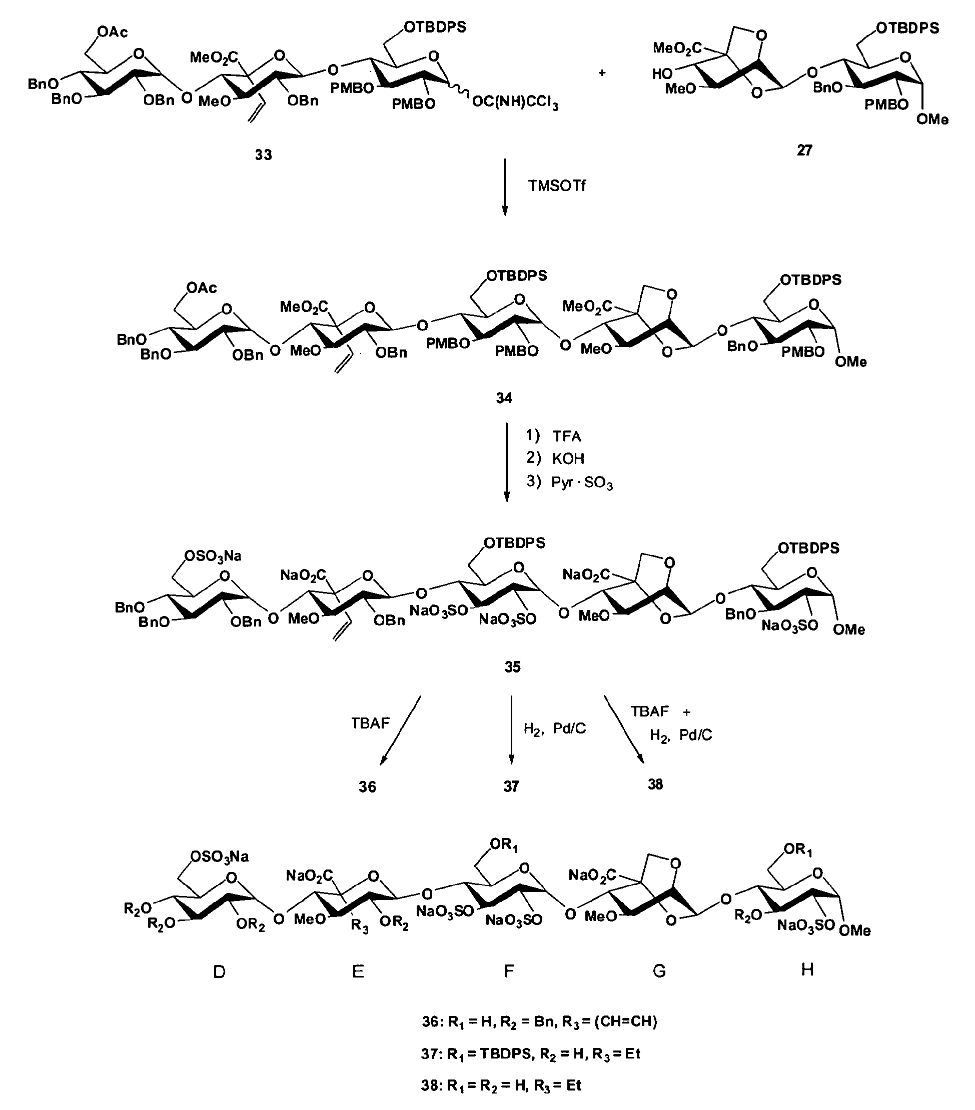

### Method A: Desilylation

Ammonium fluoride (40 molar equivalents) was added to a solution of pentasaccharide (1 molar equivalent) in methanol (70 L/mol). After stirring at room temperature for 72 hours, chromatography on a Sephadex LH-20 column (20 L/mmol) equilibrated with CH₂Cl₂/methanol/water (50:50:1) gave the desilylated product.

### Method B: Hydrogenolysis

A solution of pentasaccharide (1 molar equivalent) in 13:20 *tert*-butanol/water (250 L/mol) was stirred under hydrogen in the presence of Pd/C catalyst (10%, 2 weight equivalents) for 24 hours and filtered through Celite^{®} 45.

### Method C: Alkylation

NaH 60%/oil (18 molar equivalents) was added to a solution of pentasaccharide (1 molar equivalent) in DMF (70 L/mol) at 0 °C and the mixture was stirred for 15 minutes. After this time, the alkylating agent (18 molar equivalents) was added and the solution was stirred at room temperature for 5 hours. The resultant solution was then neutralised with ethanol, and directly poured onto a Sephadex LH-20 column (20 L/mmol) equilibrated with CH₂Cl₂/methanol/water (50:50:1) to give the alkylated product.

### Method D: Acylation

An acyl chloride (10 molar equivalents) was added to a solution of pentasaccharide (1 molar equivalent) in pyridine (70 L/mol) and DMF (70 L/mol) at 0 °C. The mixture was stirred for 16 hours at room temperature and directly poured onto a Sephadex LH-20 column (20 L/mmol) equilibrated with CH₂Cl₂/methanol/water (50:50:1) to give the acylated product.

### INTERMEDIATE PRODUCTS

The monosaccharides **4** and **21** were prepared according to procedures well known in the art. In addition, the monosaccharide donor **12** was prepared according to the procedure described in US 6,670,338 and Chem. Eur. J., 2001, 7(22), 4821.

### Preparation of the disaccharide GH 22

A mixture of **4** (92.1 mg, 0.143 mmol), **12** (84.1 mg, 0.172 mmol) and 4Å molecular sieves (220 mg) in toluene (2 mL) was stirred at room temperature for 30 minutes. The suspension was cooled at -40 °C and a 0.14 M solution of TMSOTf in toluene (0.2 ml, 0.17 eq/imidate) was added. The reaction mixture was then stirred for 90 minutes and the temperature was allowed to rise gradually to -20 °C. After this time, the reaction mixture was neutralized with triethylamine, diluted with CH₂Cl₂, filtered through Celite^{®} and concentrated.
Preparative TLC on silica gel (Toluene/AcOEt : 80/20+ 1% Et₃N) gave compound **22** (133.6 mg, 96%), which had the following properties: TLC: Rf=0.22, silica gel, toluene/AcOEt : 90/10 v/v; chemical shifts of the anomeric protons: 5.14 and 4.70 ppm; and MS (ESI⁺): *m*/*z* 993.4 [M+Na]⁺.
Disaccharide **22** was then transformed into disaccharide **27** according to the methods described in Chem. Eur. J., 2001, 7(22), 4821.

### Preparation of the disaccharide EF 28

A mixture of **17** (1.025 g, 1.467 mmol), **12** (864 mg, 1.76 mmol) and 4Å molecular sieves (2.2 g) in toluene (20 mL) was stirred at room temperature for 30 minutes. The suspension was cooled at -40 °C and a 0.29 M solution of TMSOTf in toluene (1 ml, 0.17 eq/imidate) was added. The reaction mixture was then stirred for 90 minutes and the temperature was allowed to rise gradually to -20 °C. After this time, the reaction mixture was neutralized with triethylamine, diluted with CH₂Cl₂, filtered through Celite^{®} and concentrated.
Flash column chromatography on silica gel (CH₂Cl₂/AcOEt : 93/7) gave compound **28** (1.36 g, 75%), which had the following properties: TLC: Rf=0.36, silica gel, toluene/AcOEt : 80/20 v/v; chemical shifts of the anomeric protons: 5.15 and 4.90 ppm; and MS (ESI⁺): *m*/*z* 1049.4 [M+Na]⁺.
Disaccharide **28** was then transformed into the disaccharide **31** following the methods described in Chem. Eur. J., 2001, 7(22), 4821.

### Preparation of the trisaccharide DEF 32

A mixture of **31** (402 mg, 0.367 mmol), **21** (281 mg, 0.441 mmol) and 4Å molecular sieves (1.0 g) in toluene (8 mL) was stirred at room temperature for 30 minutes. The suspension was cooled at -40 °C and a 0.29 M solution of TMSOTf in toluene (250 µl, 0.17 eq/imidate) was added. The reaction mixture was then stirred for 90 minutes and the temperature was allowed to rise gradually to -20 °C. After this time, the reaction mixture was neutralized with triethylamine, diluted with CH₂Cl₂, filtered through Celite^{®} and concentrated.
Flash column chromatography on silica gel (CH₂Cl₂/AcOEt : 95/5) gave compound **32** (436 mg, 71%), which had the following properties: TLC: Rf=0.26, silica gel, toluene/AcOEt : 80/20 v/v; chemical shifts of the anomeric protons: 5.16 , 5.08 and 4.88 ppm; and MS (ESI⁺): *m*/*z* 1592.7 [M+Na]⁺.
Trisaccharide **32** was then transformed into the trisaccharide **33** according to the methods described in Chem. Eur. J., 2001, 7(22), 4821.

### Preparation of protected pentasaccharide DEFGH 34

A mixture of **33** (189 mg, 0.113 mmol), **27** (88 mg, 0.094 mmol) and 4Å molecular sieves (500 mg) in toluene (4 mL) was stirred at room temperature for 30 minutes. The suspension was cooled at -40 °C and a 0.29 M solution of TMSOTf in toluene (64 µl, 0.17 eq/imidate) was added. The reaction mixture was then stirred for 90 minutes and the temperature was allowed to rise gradually to -20 °C. After this time, the reaction mixture was then neutralized with triethylamine, diluted with CH₂Cl₂, filtered through Celite^{®} and concentrated.
Flash column chromatography on silica gel (CH₂Cl₂/AcOEt : 95/5) gave compound **34** (168 mg, 73%), which had the following properties: TLC: Rf=0.33, silica gel, CH₂Cl₂/AcOEt : 90/10 v/v; chemical shifts of the anomeric protons: 5.50 , 5.28, 5.16 , 4.98 and 4.72 ppm; MS (ESI⁺): m/z 1592.7 [M+Na]⁺.

### Preparation of pentasaccharide DEFGH 35

Pentasaccharide **34** (115 mg, 50µmol) was dissolved at 0°C in 18.4 ml of a mixture CH₂Cl₂/TFA (99/1). The solution was stirred at room temperature during 12 hours and diluted with CH₂Cl₂.
After washing with aqueous saturated NaHCO₃ solution, the organic layer was dried on MgSO₄, concentrated and purified by chromatography on silica gel (CH₂Cl₂/MeOH : 95/5) to give 76 mg of an intermediate pentasaccharide which was dissolved in 5.4 ml of a mixture THF/MeOH (2/1). Then, 1.7 ml of a 2 M aqueous KOH solution were added dropwise at 0°C and the mixture was stirred 2 hours at room temperature. After stirring, the reaction mixture was acidified with ion-exchange resin Dowex 50WX8-200, filtered and concentrated to dryness.
The resultant pentasaccharide was dissolved in 7.6 ml of dry pyridine and sulfurtrioxide pyridine complex (181 mg, 1 mmol) was added. The mixture was heated at 55°C with protection of light for 18 hours.
After cooling to 0°C, the solution was neutralized with MeOH and an aqueous saturated NaHCO₃ solution. The reaction mixture was directly poured onto Sephadex LH20 (dichloromethane/methanol : 1/1+ water 1%) to give the *O*-sulfonated pentasaccharide **35** (60 mg, 53%), which had the following properties: chemical shifts of the anomeric protons: 5.47, 5.31, 5.16, 4.71 and 4.67 ppm; MS (ESI⁻): chemical mass=2316.37; experimental mass=2318.3.

### Preparation of pentasaccharide DEFGH 36

Pentasaccharide **35** (53 mg, 21.6 µmol) was desilylated according to 'Method A: Desilylation' to give pentasaccharide **36** (37 mg, 86%), which had the following properties: chemical shifts of the anomeric protons: 5.39, 5.29, 5.17, 4.68 and 4.66 ppm; and MS (ESI⁻): chemical mass=1838.25; experimental mass=1839.5.

### Preparation of pentasaccharide DEFGH 37

Pentasaccharide **35** (42 mg, 17.1 µmol) was hydrogenolysed according to 'Method B: Hydrogenolysis' to give pentasaccharide **37** (35.7 mg, 85%), which had the following properties: chemical shifts of the anomeric protons: 5.41, 5.29, 5.16, 4.67 and 4.65 ppm; and MS (ESI⁻): chemical mass=1728.33; experimental mass=1729.4.

### Preparation of pentasaccharide DEFGH 38

Pentasaccharide **36** (37 mg, 18.6 µmol) was hydrogenolysed according to 'Method B: Hydrogenolysis' to give pentasaccharide **38** (21.5 mg, 83%), which had the following properties: chemical shifts of the anomeric protons: 5.31, 5.22, 5.03, 4.69 and 4.61 ppm; and MS (ESI⁻): chemical mass=1252.09; experimental mass=1253.1.

### EXAMPLES

### Example 1

### Synthesis of Methyl O-(2,3,4-tri-O-butyl-6-O-sulfo-α-D-glucopyranosyl)-(1→ 4)-O-(2-O-butyl-5-C-ethyl-3-O-methyl-β-D-glucopyranosyluronic acid)-(1→ 4)-O-(6-O-butyl-2,3-di-O-sulfo-α-D-glucopyranosy)-(1→ 4)-O-(2,6-anhydro-5-C-carboxy-3-O-methyl-β-D-mannopyranosyl)-(1→ 4)-3,6-di-O-butyl-2-O-sulfo-α-D-glucopyranoside, hexasodium salt 39.

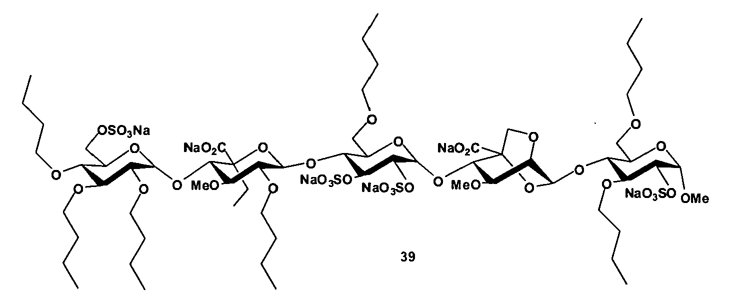

Pentasaccharide **38** (10 mg, 7.2 µmol) was alkylated with 1-bromobutane according to 'Method C: Alkylation' to give pentasaccharide **39** (10.4 mg, 81%), which had the following properties: chemical shifts of the anomeric protons: 5.42, 5.38, 5.15, 4.74 and 4.68 ppm; and MS (ESI⁻): chemical mass=1782.47; experimental mass=1783.4.

### Example 2

### Synthesis of Methyl O-(2,3,4-tri-O-nonanoyl-6-O-sulfo-α-D-glucopyranosyl)-(1→ 4)-O-(5-C-ethyl-3-O-methyl-2-O-nonanoyl-β-D-glucopyranosyluronic acid)-(1→ 4)-O-(6-O-nonanoyl-2,3-di-O-sulfo-α- D-glucopyranosyl)-(1→ 4)-O-(2,6-anhydro-5-C-carboxy-3-O-methyl-β-D-mannopyranosyl)-(1→ 4)-3 ,6-di-O-nonanoyl-2-O-sulfo-α-D-glucopyranoside, hexasodium salt 40.

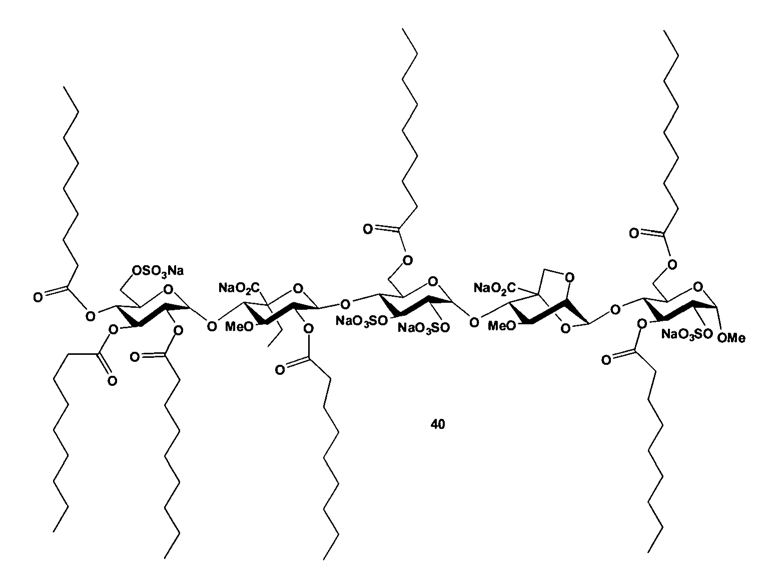

Pentasaccharide **38** (10 mg, 7.2 µmol) was acylated with 2-nonanoyl chloride according to 'Method D: Acylation' to give pentasaccharide **40** (13.4 mg, 78%), which had the following properties: chemical shifts of the anomeric protons: 5.54, 5.52, 5.21, 4.86 and 4.72 ppm; and MS (ESI⁻): chemical mass=2370.87; experimental mass=2372.1.

### Example 3

### Synthesis of Methyl O-(6-O-sulfo-2,3,4-tri-O-(4-tert-butylbenzyl)-α-D-glucopyranosyl)-(1→ 4)-O-(5-C-ethyl-3-O-methyl-2-O-(4-tert-butylbenzyl)-β-D-glucopyranosyluronic acid)-(1→ 4)-O-(6-O-cyclopentanepropionyl-2,3-di-O-sulfo-α-D-glucopyranosyl)-(1→ 4)-O-(2,6-anhydro-5-C-carboxy-3-O-methyl-β-D-mannopyranosyl)-(1- 4)-6-O-cyclopentane propionyl-2-O-sulfo-3-O-(4-tert-butylbenzyl)-α-D-glucopyranoside, hexasodium salt 41.

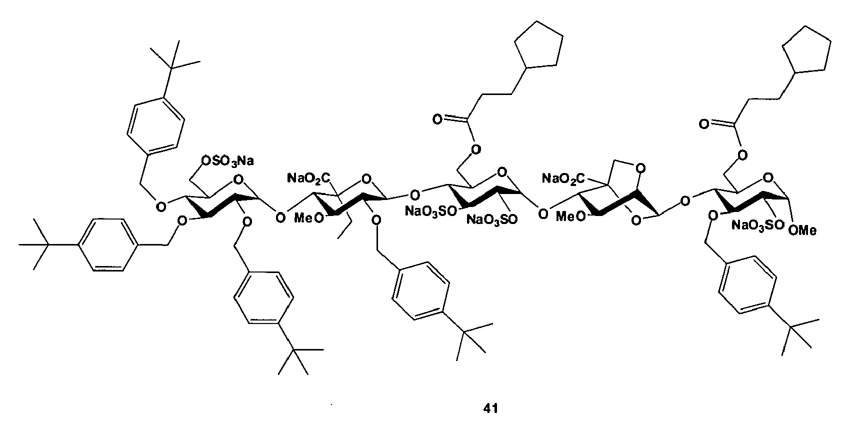

Pentasaccharide **37** (19 mg, 10.1 µmol) was alkylated with 4-*tert*-butylbenzyl chloride according to 'Method C: Alkylation'. The resulting compound was desilylated in the manner described in 'Method A: Desilylation', and acylated with cyclopentanepropionyl chloride according to 'Method D: Acylation' to give pentasaccharide **41** (12.7 mg, 81%), which had the following properties: chemical shifts of the anomeric protons: 5.52, 5.48, 5.26, 4.89 and 4.68 ppm; and MS (ESI⁻): chemical mass=2230.82; experimental mass=2231.9.

### Example 4

### Synthesis of Methyl O-(2,3,4-tri-O-hexanoyl-6-O-sulfo-α-D-glucopyranosyl)-(1→ 4)-O-(5-C-ethyl-2-O-hexanoyl-3-O-methyl-β-D-glucopyranosyluronic acid)-(1→ 4)-O-(6-O-(2,2-dimethylpropyl)-2,3-di-O-sulfo-α-D-glucopyranosyl)-(1-4)-O-(2,6-anhydro-5-C-carboxy-3-O-methyl-β-D-mannopyranosyl)-(1→ 4)-6-O-(2,2-dimethylpropyl)-3-O-hexanoyl-2-O-sulfo-α-D-glucopyranoside, hexasodium salt 42.

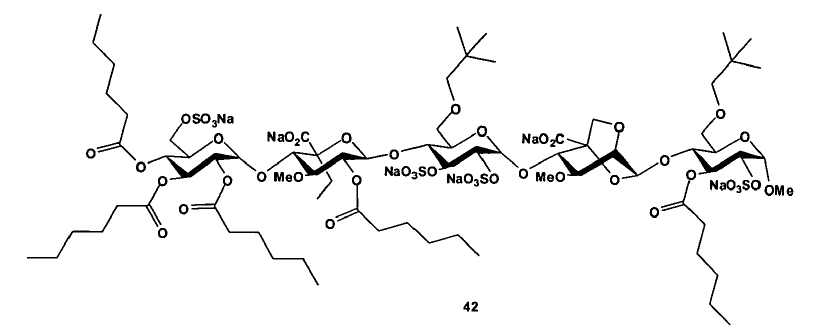

Pentasaccharide **36** (18 mg, 9.1 µmol) was alkylated with 1-bromo-2,2-dimethylpropane according to 'Method C: Alkylation'. The resulting compound was hydrogenolysed in a manner as described in 'Method B: Hydrogenolysis', and acylated with hexanoyl chloride according to 'Method D: Acylation' to give pentasaccharide **42** (12.3 mg, 67%), which had the following properties: chemical shifts of the anomeric protons: 5.56, 5.49, 5.26, 4.89 and 4.71 ppm; and MS (ESI⁻): chemical mass=1882.61; experimental mass=1883.7.

### Example 5

### Synthesis of Methyl O-(2,3,4-tri-O-(4-chlorobenzyl)-6-O-sulfo-α-D-glucopyranosyl)-(1→ 4)-O-(2-O-(4-chlorobenzyl)-5-C-ethyl-3-O-methyl-β-D-glucopyranosyluronic acid)-(1→ 4)-O-(2,3-di-O-sulfo-α-D-glucopyranosyl)-(1→ 4)-O-(2,6-anhydro-5-C-carboxy-3-O-methyl-β-D-mannopyranosyl)-(1→ 4)-3-O-(4-chlorobenzyl)-2-O-sulfo-α-D-glucopyranoside, hexasodium salt 43.

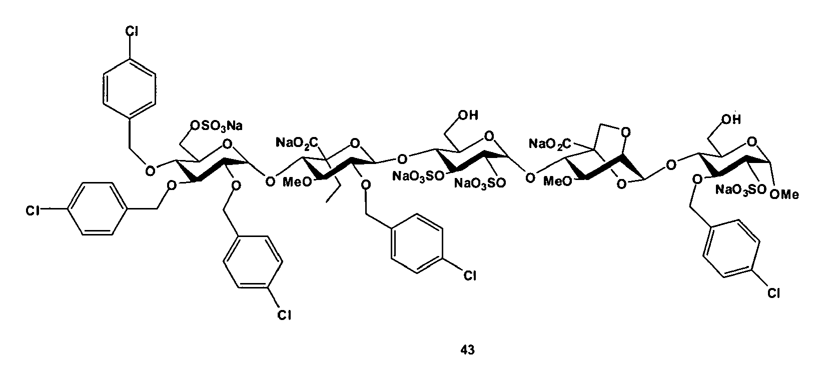

Pentasaccharide **37** (16 mg, 8.6 µmol) was alkylated with 4-chlorobenzyl chloride according to 'Method C: Alkylation'. The resulting compound was desilylated in the manner described in 'Method A: Desilylation' to give pentasaccharide **43** (12.4 mg, 72%), which had the following properties: chemical shifts of the anomeric protons: 5.67, 5.62, 5.22, 4.89 and 4.64 ppm; and MS (ESI⁻): chemical mass=2010.07; experimental mass=1911.3.

### Example 6

### Synthesis of Methyl O-(6-O-sulfo-α-D-glucopyranosyl)-(1→ 4)-O-(5-C-ethyl-3-O-methyl-β-D-glucopyranosyluronic acid)-(1→ 4)-O-(6-O-(deoxycholoyl)-2,3-di-O-sulfo-α-D-glucopyranosyl)-(1→ 4)-O-(2,6-anhydro-5-C-carboxy-3-O-methyl-β-D-mannopyranosyl)-(1→ 4)-6-O-(deoxycholoyl)-2-O-sulfo-α-D-glucopyranoside, disodium and tetra-tripropyl ammonium salt 44.

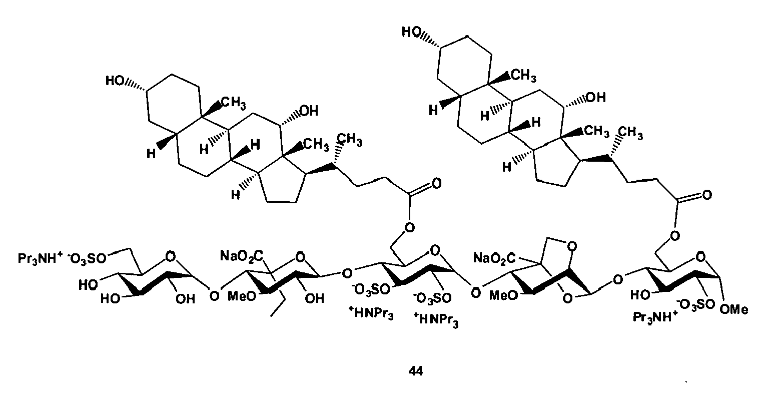

Pentasaccharide **36** (18 mg, 9.1 µmol) was acylated with deoxycholoyl chloride according to 'Method D: Acylation'. The resulting compound was hydrogenolysed in the manner described in 'Method B: Hydrogenolysis'. The resulting pentasaccharide sodium salt was dissolved in water and an aqueous solution of tripropyl ammonium chloride (4 equivalents) was added. The mixture was stirred at room temperature for 16 hours. The solution was then loaded on top of a Sephadex G25F column (50 mL) equilibrated with water. The fractions containing the compound were collected and concentrated to give the pentasaccharide-tripropyl ammonium ion complex **44** (10.7 mg, 45%), which had the following properties: chemical shifts of the anomeric protons: 5.68, 5.62, 5.22, 4.89 and 4.64 ppm; and MS (ESI⁻): chemical mass=2000.66; experimental mass=2001.7.

### BIOLOGICAL TESTING

It will be understood that a variety of assays are suitable for testing the biological activity of the compounds of the present invention. However, suitable methods for testing the biological activity of the compounds of the present invention are listed below.

### Example - Quantification of the compounds of the present invention in rat plasma

Rat plasmatic concentration of the compounds of the present invention was determined by its anti-factor Xa activity using COATEST^{®} Heparin, which allows the specific determination of the anti-factor Xa activity of heparin. The COATEST^{®} Heparin Kit was from Chromogenix, Montpellier. Absorbance measures were performed with the Multiskan Ascent absorbance plate reader (Thermo Labsystems (Helsinki, Finland) and normal rat plasma was from Charles River Laboratories.

Rat blood (9 volumes) was mixed with sodium citrate (1 volume) and preferably cooled immediately on ice to minimise release of heparin antagonists from blood cells. As soon as possible after blood collection, the sample was subjected to a centrifuge at 3000 x g for 10 minutes at low temperature (the plasma is, typically, stable for 24 hours at temperatures below 8 °C).

A standard curve was performed by a two step procedure for dilution of the compounds described in the present invention. First, the compounds were diluted in physiological serum and then 100-fold dilution was performed in working Buffer (Tris 50 mM, pH 8.4). Standards containing the defined concentration of the compounds of the present invention and the tested plasmas were mixed with working buffer solution and antithrombin and finally incubated at 37 °C for 3 minutes. The dosage of antifactor Xa activity was then performed by the addition of factor Xa in excess followed by a 30 second incubation at 37 °C. The factor Xa was neutralized in proportion to the amount of the compounds of the present invention used, the remaining amount of factor Xa hydrolyses the chromogenic substrate S-2222 thus liberate a chromogenic group. The reaction was finally stopped with acetic acid 20 %.

Following completion of the reaction, a blank was composed of sample blank, acetic acid and water and the absorbance was read at 405 nm. The corrected absorbencies were plotted against their concentrations of the compounds of the present invention. The obtained standard curve allowed the concentration of the compounds of the present invention in the plasma to be calculated.

### Pharmacokinetic profile determination

The compounds of the present invention were prepared in solution ready for oral and intravenous administration, and the doses were varied. In humans, oral administration is the preferred route of administration.

The pharmacokinetics of the compounds of the present invention were investigated in female Wistar Han rats after a single administration by oral gavage vs intravenous route using a bioassay which utilised the inherent factor-Xa inhibitory activity of the compounds as described above as a means of detection and determination of plasma compound concentration expressed in µg/ml (microg/ml).

The blood of the animals was sampled for 48 hours. For each route of administration (*i.e.* the intravenous and oral routes), the maximum plasma concentration of the compound (Cₘₐₓ) and the time (Tₘₐₓ) to reach maximum plasma concentration of the compound were determined from the plasma concentration data vs time data. After administration of the compounds of the present invention, the area under the plasma concentration vs time curve up to ∞ (AUC_{0→ ∞}) was calculated using the trapezoid rule.

In addition to the above, the bioavailability of the compounds of the present invention was calculated as the ratio of the AUC (oral)/AUC (intravenous). Each dose and route of administration was tested on a group composed of 6 to 8 animals and the results were given as mean +/- the standard deviation.

The apparent volume of distribution was small indicating restriction mainly to the blood compartment, as would be expected for this type of molecule which displays high and specific affinity for plasma anti-thrombin.

The compounds of the present invention were administered via an oral gavage, which was carefully passed down the oesophagus into the stomach. The gavage was made from stainless steel with a blunt end to avoid causing injury to the tissue surface. After administration of a single oral dose in rats, the time to reach peak plasma concentration of the compound (Tₘₐₓ) and the maximum plasma concentration of the compound (Cₘₐₓ) was measured.

## Claims

1. A compound comprising an oligosaccharide of Formula (I): 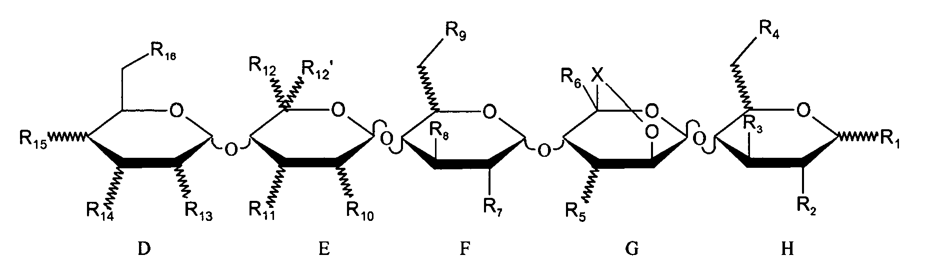 wherein:
R₂, R₇, R₈ and R₁₆ are independently selected from the group consisting of: OSO₃H and NHSO₃H;
R₆ and R₁₂ are each COOH;
R₁, R₃, R₄, R₅, R₉, R₁₀, R₁₁, R₁₃, R₁₄ and R₁₅ are independently selected from the group consisting of: OH, OSO₃H, NH₂, O-alkyl, O-acyl, O-alkenyl, O-alkynyl, O-aryl, O-heteroaryl, O-heterocyclyl, O-aminoalkyl, O-alkylaryl, O-alkylheteroaryl, O-alkylheterocyclyl;
provided at least one of R₃, R₄, R₉, R₁₀, R₁₃, R₁₄ and R₁₅ is independently selected from the group consisting of: NH₂, O-(C₄₋₃₀-alkyl), O-(C₄₋₃₀-acyl), O-alkenyl, O-alkynyl, O-aryl, O-heteroaryl, O-heterocyclyl, O-aminoalkyl, O-alkylaryl, O-alkylheteroaryl, O-alkylheterocyclyl;
R_{12'} is selected from the group consisting of: H and alkyl;
X is selected from the group consisting of: CH₂ and CH₂CH₂; and
wherein any of R₃, R₄, R₉, R₁₀, R₁₃, R₁₄ and R₁₅ are independently optionally substituted with one or more groups independently selected from alkyl, alkoxyalkyl, alkoxyaryl, alkynyl, heteroaryl, aryl, arylalkyl, alkaryl, COOH, COOalkyl, SH, S-alkyl, SO₂H, SO₂alkyl, SO₂aryl, SO₂alkaryl, P(OH)(O)₂, halo, haloalkyl, perhaloalkyl, OH, O-alkyl, =O, NH₂, =NH, NHalkyl, N(alkyl)₂, =Nalkyl, NHC(O)alkyl, C(O)NH₂, C(O)NHalkyl, C(O)N(alkyl)₂, C(O)NHaryl, NO₂, CN, SO₂, SO₂NH₂, C(O)H, C(O)alkyl;
or a salt, solvate or prodrug thereof.

2. The compound, salt, solvate or prodrug of claim 1 wherein R₁, R₅ and R₁₁ are each O-alkyl.

3. The compound, salt, solvate or prodrug of any one of the claims 1 and 2 wherein:
R₃ is OSO₃H;
R₁₀ is OCH₃;
R₁₃ is NH₂; and
R₄, R₉, R₁₄ and R₁₅ are each OH.

4. The compound, salt, solvate or prodrug of the claims 1 and 2 wherein:
R₃, R₄, R₉, R₁₀, R₁₃, R₁₄ and R₁₅ are independently selected from the group consisting of: OH, OSO₃H, NH₂, O-(C₄₋₃₀-alkyl)_{,} O-(C₄₋₃₀-acyl), O-alkenyl, O-alkynyl, O-aryl, O-heteroaryl, O-heterocyclyl, O-aminoalkyl, O-alkylaryl, O-alkylheteroaryl, O-alkylheterocyclyl;
wherein any of R₃, R₄, R₉, R₁₀, R₁₃, R₁₄ and R₁₅ are independently optionally substituted with one or more groups independently selected from alkyl, alkoxyalkyl, alkoxyaryl, alkynyl, heteroaryl, aryl, arylalkyl, alkaryl, COOH, COOalkyl, SH, S-alkyl, SO₂H, SO₂alkyl, SO₂aryl, SO₂alkaryl, P(OH)(O)₂, halo, haloalkyl, perhaloalkyl, OH, O-alkyl, =O, NH₂, =NH, NHalkyl, N(alkyl)₂, =Nalkyl, NHC(O)alkyl, C(O)NH₂, C(O)NHalkyl, C(O)N(alkyl)₂, NO₂, CN, SO₂ SO₂NH₂ C(O)H, C(O)alkyl and C(O)NHaryl.

5. The compound, salt, solvate or prodrug of any one of the claims 1 to 4 wherein monosaccharide unit G of the oligosaccharide has the following conformation:

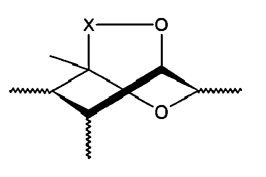

6. The compound, salt, solvate or prodrug of any one of the claims 1 to 5 wherein monosaccharide units D, E, F and H of the oligosaccharide have the D-gluco stereochemistry as follows:

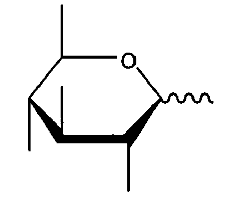

7. The compound, salt, solvate or prodrug of any one of the claims 1 to 6 wherein monosaccharide unit G of the oligosaccharide has the following stereochemistry:

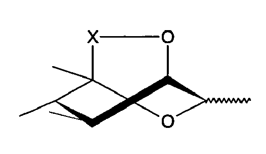

8. The compound, salt, solvate or prodrug of any one of the claims 1 to 7 wherein R₁, R₅ and R₁₁ are each OMe.

9. The compound, salt, solvate or prodrug of any one of any one of the claims 1 to 8 wherein R₂, R₇, R₈ and R₁₆ are each OSO₃H.

10. The compound, salt, solvate or prodrug of any one of the claims 1 to 9 wherein X is CH₂.

11. The compound, salt, solvate or prodrug of any one of the claims 1 to 10 wherein R₁₂, is CH₂CH₃.

12. The compound, salt, solvate or prodrug of any one of the claims 4 to 11 wherein R₃, R₄, R₉, R₁₀, R₁₃, R₁₄ and R₁₅ are independently selected from: OH, OSO₃H, NH₂, O-(C₄₋₃₀-alkyl), O-(C₄₋₃₀-acyl), O-heterocyclyl, O-aryl, O-alkylaryl;
wherein any of R₃, R₄, R₉, R₁₀, R₁₃, R₁₄ and R₁₅ are independently optionally substituted with one or more groups independently selected from halo, haloalkyl, perhaloalkyl, OH, O-alkyl, =O, alkyl, alkoxyalkyl, alkoxyaryl, alkynyl, arylalkyl, alkaryl, heteroaryl and aryl.

13. The compound, salt, solvate or prodrug of any one of the claims 1 to 12 wherein any of R₃, R₄, R₉, R₁₀, R₁₃, R₁₄ and R₁₅ are independently selected from: O-butyl, nonanoyl, (4-tert-butyl)benzyloxy, 3-cyclopentylpropanoyl, hexanoyl, 2,2-dimethylpropyloxy, 4-chlorobenzyloxy, OH and deoxycholoyl.

14. The compound, salt, solvate or prodrug of claim 1 wherein the oligosaccharide is of Formula (II):

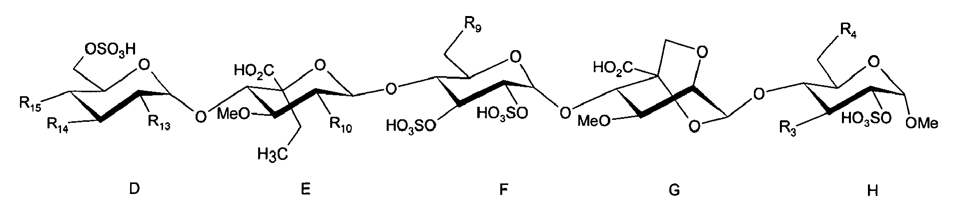

15. The compound, salt, solvate or prodrug of claim 14 wherein R₁₀ is OCH₃.

16. The compound, salt, solvate or prodrug of any one of the claims 1 to 15 wherein R₃ is OSO₃H.

17. The compound, salt, solvate or prodrug of claim 14 wherein:
R₃, R₄, R₉, R₁₀, R₁₃, R₁₄ and R₁₅ are each O-butyl.

18. The compound, salt, solvate or prodrug of claim 14 wherein:
R₃, R₄, R₉, R₁₀, R₁₃, R₁₄ and R₁₅ are each nonanoyl.

19. The compound, salt, solvate or prodrug of claim 14 wherein:
R₃, R₁₀, R₁₃, R₁₄ and R₁₅ are each (4-tert-butyl)benzyloxy ; and
R₄, R₉ are each 3-cyclopentylpropanoyl.

20. The compound, salt, solvate or prodrug of claim 14 wherein:
R₃, R₁₀, R₁₃, R₁₄ and R₁₅ are each hexanoyl; and
R₄, R₉ are each 2,2-dimethylpropyloxy.

21. The compound, salt, solvate or prodrug of claim 14 wherein:
R₃, R₁₀, R₁₃, R₁₄ and R₁₅ are each 4-chlorobenzyloxy; and
R₄, R₉ are each OH.

22. The compound, salt, solvate or prodrug of claim 14 wherein:
R₃, R₁₀ R₁₃, R₁₄ and R₁₅ are each OH; and
R₄, R₉ are each deoxycholoyl.

23. The salt of any preceding claim wherein the counter-ion is selected from the group consisting of: sodium and potassium.

24. A pharmaceutical composition comprising a compound, salt, solvate or prodrug according to any preceeding claim and a pharmaceutically acceptable diluent or carrier.

25. A method of making a pharmaceutical composition according to claim 24, comprising mixing said compound, salt, solvate or pro-drug with a pharmaceutically acceptable diluent or carrier.

26. A compound, salt, solvate or pro-drug according to any of claims 1 to 23, for use in therapy.

27. The use of a compound, salt, solvate or prodrug as described in any one of the claims 1 to 23 in the manufacture of a medicament for the treatment of a blood clotting disorder.

28. A method of treating a blood clotting disorder in a human or animal subject comprising administering to the human or animal subject a therapeutically effective amount of a compound, salt, solvate or prodrug as defined in any one of the claims 1 to 23.

29. The use or method of any one of the claims 27 and 28, wherein the medicament is orally administered.

30. The use or method of any one of the claims 27 to 29, wherein the blood clotting disorder is selected from: deep vein thromboembolism including deep vein thrombosis and pulmonary embolism, post surgical prophylaxis of deep venous thrombosis, coronary syndromes, myocardial infarcation and stroke.
